# FASCICULE DE BREVET EUROPEEN

(11) **EP 1 265 645 B1**
(45) Date de publication et mention de la délivrance du brevet: **18.08.2004**
(21) Numéro de dépôt: 01917168.5
(22) Date de dépôt: 16.03.2001
(51) Int. Cl.: A61L 9/012

(54) **PREPARATION PARFUMEE SOLIDE SOUS FORME DE MICROBILLES ET SON UTILISATION**
FESTE PARFÜMZUSAMMENSETZUNG IN FORM VON MIKROKUGELN UND DEREN VERWENDUNG
SOLID PERFUMED PREPARATION IN THE FORM OF MICROBEADS AND USE THEREOF

(30) Priorité: 20.03.2000 FR 0003535
(43) Date de publication de la demande: 18.12.2002
(73) Titulaire: V.Mane Fils, 06620 Bar sur Loup (FR)
(72) Inventeur: MANE, Jean, F-06130 Grasse (FR); BLEUEZ, Loic, F-06700 Saint Laurent du Var (FR); DELPECH, Guy, F-06130 Grasse (FR); HOC, Nathalie, F-06130 Grasse (FR); HANNETEL, Jean-Michel, F-06130 Grasse (FR); DAILLAND, Pascal, F-06130 Grasse (FR)
(74) Mandataire: Vercaemer, Laurence
(86) Numéro de dépôt international: PCT/FR2001/000798
(87) Numéro de publication internationale: WO 2001/070283

(56) Documents cités:
- EP-A- 0 317 658
- US-A- 3 985 298
- US-A- 4 610 927
- US-A- 4 906 488

## Description

La présente invention concerne des préparations parfumées solides sous forme de microbilles. Ces préparations permettent de libérer une matière première aromatique volatile, voire très volatile, de façon contrôlée et sur une longue durée (rémanence), et peuvent être utilisées même dans un milieu agressif, donc avec beaucoup de supports cosmétiques usuels ou dans des produits ménagers tels que des détergents.

On utilise des préparations parfumées, c'est-à-dire des préparations comprenant des matières premières aromatiques volatiles, depuis longtemps et dans de nombreux produits, en particulier des produits cosmétiques et des produits d'entretien.

Néanmoins, on cherche encore à conserver ou améliorer l'action odorante de ces préparations, en particulier la rémanence de l'odeur. En effet, dans les formulations classiques, les matières premières aromatiques ont une rémanence très courte sur la peau ou les cheveux, en particulier à cause de leur volatilité parfois très élevée. Or il est important, particulièrement en cosmétique, mais également dans les produits ménagers, de disposer de préparations dont l'odeur agréable subsiste longtemps.

Par ailleurs, de nombreux supports utilisés dans des compositions cosmétiques ou des produits ménagers sont des milieux agressifs, qui peuvent dégrader ou altérer les matières premières aromatiques, qui sont des composés fragiles. C'est le cas en particulier de compositions à base de thioglycolate, d'alpha hydroxyacides, de peroxydes d'hydrogène, de composés aminés, d'ammoniaque, de dihydroxyacétone, de filtres solaires, de sels d'aluminium ou de bases fortes. Il est donc tout aussi important de pouvoir disposer de matières premières aromatiques volatiles sous une forme telle qu'elles conservent leurs propriétés odorantes, en particulier la rémanence de l'odeur, même dans de tels milieux agressifs. Ceci permet de les utiliser en association avec un plus grand nombre de supports cosmétiques ou de produits ménagers usuels, et donc dans un plus grand nombre d'applications dans l'industrie cosmétique et l'industrie des produits ménagers.

Pour répondre à ce souci sans cesse croissant de conservation de l'odeur dans la préparation ou composition finale et d'augmentation de la rémanence de cette odeur, la Demanderesse a donc cherché à développer une préparation intégrant un véhicule de transport des molécules ou matières premières aromatiques volatiles qui soit en mesure de les protéger du milieu extérieur et de favoriser la conservation et la rémanence de l'odeur.

Elle a trouvé que ces objectifs pouvaient être atteints grâce à l'association de la ou des matières premières aromatiques volatiles avec un ou des excipients cosmétiques fusibles, et à une présentation sous forme de microbilles solides stables et à rémanence élevée.

En fonction du choix des composés, la préparation selon l'invention permet une libération de matières premières aromatiques volatiles qui est deux à vingt fois plus longue que celle des mêmes matières premières aromatiques conditionnées de façon classique, par exemple sous forme de solution alcoolique à libération immédiate. Ainsi, la préparation selon l'invention présente une rémanence élevée, c'est-à-dire qu'elle permet d'améliorer significativement la rémanence d'un parfum, par exemple dans un hydrogel ou dans un autre support cosmétique ou ménager.

Un autre avantage de la préparation selon l'invention est qu'elle permet l'utilisation de matières premières aromatiques volatiles jugées non stables dans un milieu très agressif et qui, actuellement, ne sont donc pas utilisées du tout ou pas de façon efficace. En effet, lorsqu'elles sont conditionnées de façon classique, elles perdent leur propriétés odorantes et donc leur intérêt. La possibilité de les utiliser dans de tels milieux a pour conséquence d'accroître la créativité, en terme d'axes olfactifs, concernant le parfumage de supports cosmétiques ou de produits ménagers difficiles : on aura un choix beaucoup plus grand de matières premières aromatiques et de supports cosmétiques ou de produits ménagers utilisables.

On connaît déjà des présentations de produits chimiques sous forme de microbilles. Cependant, il s'agit souvent de produits minéraux, tels que des engrais, qui sont peu sensibles à la chaleur ou à l'humidité, donc peu fragiles, et qui sont mis sous cette forme de microbilles simplement pour une plus grande facilité d'emploi.

On connaît également des présentations de principes actifs pharmaceutiques sous forme de microbilles. Les problèmes soulevés dans ce domaine sont cependant particuliers et différents des problèmes soulevés par l'utilisation de matières premières volatiles. En effet, il s'agit dans le domaine pharmaceutique de modifier la cinétique de libération d'un principe actif, sans modifier ses propriétés physico-chimiques et donc son activité pharmacologique. Le problème de la volatilité de la matière première n'est donc pas abordé.

Ainsi, les présentations de produits sous forme de microbilles solides de l'art antérieur sont-elles conçues pour une simple facilité d'utilisation, ou pour modifier la cinétique de libération d'un principe actif faisant partie d'une composition pharmaceutique. Les problèmes liés à la volatilité des matières premières, et à leur sensibilité au milieu, ne sont ni posés ni a fortiori résolus.

Dans le cas de préparations parfumées, la présentation sous forme de microbilles solides permet à la fois de mettre en forme des composés volatils, de protéger ces composés des phénomènes d'oxydation et autres altérations, d'obtenir une rémanence élevée, et de permettre l'utilisation de la préparation avec des supports cosmétiques ou de produits ménagers usuels même s'ils sont agressifs. De plus, les microbilles ont également pour avantage d'avoir un impact visuel intéressant, de présenter une distribution régulière quant au diamètre et à la forme, et de pouvoir faire cohabiter des populations granulométriques différentes.

L'invention a donc pour objet une préparation parfumée solide à rémanence améliorée sous forme de microbilles telle que définie dans la revendication 1, notamment, comprenant une matière première aromatique volatile ou un mélange de matières premières aromatiques volatiles, et un ou plusieurs excipients fusibles. Elle a également pour objet le procédé de fabrication d'une telle préparation tel que défini dans la revendication 15, et une formulation cosmétique ou de produit ménager comprenant une telle préparation (voir revendication 12).

Le terme « matière première aromatique volatile » désignera dans la présente demande indistinctement une matière première aromatique volatile ou un mélange de matières premières aromatiques volatiles.

Un excipient fusible est un excipient qui passe à l'état liquide sous l'effet de la chaleur. Les excipients fusibles utilisables sont les excipients fusibles de qualité cosmétique ou adaptés à une utilisation dans des produits ménagers, c'est-à-dire, à titre d'exemples non limitatifs, des détergents (poudre à lessiver et liquide), des produits assouplissants, des produits pour le traitement des textiles.

La matière première aromatique volatile utilisée dans la préparation selon l'invention peut être toute molécule aromatique c'est-à-dire odorante, mais n'est pas nécessairement une molécule aromatique au sens chimique du terme. Par exemple, une matière première aromatique pourra être choisie parmi :
- les hydrocarbures aromatiques, terpéniques et/ou sesquiterpéniques, et plus particulièrement les huiles essentielles contenant ces molécules, plus particulièrement encore les huiles essentielles d'agrumes (citron, orange, pamplemousse, bergamote), de noix de muscade,
- les alcools aromatiques, et plus particulièrement l'alcool benzylique, l'alcool phényléthylique, l'alcool phénylpropylique,
- les alcools non aromatiques primaires ou secondaires ou tertiaires, saturés ou insaturés, cycliques ou acycliques, et plus particulièrement le linalol, le citronellol, le géraniol, le nérol, le 2,6-diméthyl-7-octèn-2-ol, le terpinéol, les alcools alicycliques gras dont la chaîne comporte de 4 à 10 atomes de carbone,
- les aldéhydes, et plus particulièrement les aldéhydes gras saturés ou insaturés alicycliques dont la chaîne carbonée comporte de 4 à 12 atomes de carbone, les aldéhydes aromatiques tels que l'aldéhyde cinnamique, l'aldéhyde alpha amyl cinnamique, l'aldéhyde alpha héxyl cinnamique, le butylphényl méthylpropional (connu sous la marque Lilial), les aldéhydes aromatiques phénoliques tels que la vanilline et l'éthyl vanilline,
- les phénols, et plus particulièrement les phénols aromatiques tels que l'eugénol, l'isoeugénol ainsi que leurs éthers méthyliques,
- les acides carboxyliques, principalement sous leur forme suivante :
   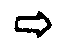 les esters, et plus particulièrement les esters acétiques de l'alcool benzylique, du géraniol, du citronellol, du nérol, du terpinéol, du bornéol, du linalol,
   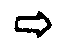 les esters des acides aromatiques tels que les benzoates et les salicylates ainsi que les cinnamates estérifiés avec les alcools de la série aliphatique possédant une chaîne carbonée de 1 à 6 atomes de carbone,
   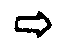 les acides-phénol aromatiques, principalement sous leur forme lactonique /aromatique tels que la coumarine et la dihydrocoumarine,
   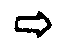 les acides alcools carboxyliques sous leur forme lactonique, et plus particulièrement les gamma octa, gamma nona, gamma undéca, gamma dodéca, delta déca, delta undéca, delta dodéca lactones sous leur forme saturée ou insaturée ainsi que les lactones macrocycliques dont la chaîne carbonée contient de 12 à 16 atomes de carbone,
- les éthers et acétals aromatiques et/ou non aromatiques sous leur forme acyclique ou cyclique, et plus particulièrement les acétals des aldéhydes à chaîne carbonée possédant de 4 à 10 atomes de carbone ainsi que des éthers cycliques furaniques ou pyraniques substitués ou non substitués,
- les hétérocycles contenant l'atome d'azote, et plus particulièrement les dérivés indolés, ainsi que les hétérocycles contenant deux atomes d'azote, et plus particulièrement les dérivés de la série des pyrazines,
- les cétones, en particulier les cétones aromatiques telles que la 4-(p-hydroxyphényl}-2-butanone, et les cétones non aromatiques saturées ou insaturées, cycliques ou non cycliques,
- les sulfures, disulfures et mercaptans aromatiques ou non aromatiques.

La préparation selon l'invention comprend en outre un ou plusieurs excipients fusibles de qualité cosmétique ou adaptés à une utilisation dans des produits ménagers.

Les excipients fusibles utilisables sont les excipients appropriés pour permettre à la fois la protection de la matière première aromatique, la libération contrôlée et la mise en forme sous forme de microbille. Ils peuvent être choisis parmi :
- les alcools gras tels que l'alcool cétylique, l'alcool béhénylique, l'alcool cétostéarylique, l'alcool stéarylique,
- les acides gras tels que l'acide stéarique, l'acide myristique, l'acide béhénique, l'acide palmitique,
- les esters de glycérol tels que : le palmitostéarate de glycérol, le stéarate de glycérol, le tribéhénate de glycérol,
- les huiles hydrogénées telles que l'huile de ricin hydrogénée, l'huile de palme hydrogénée, l'huile de maïs hydrogénée, l'huile de jojoba hydrogénée,
- le sels d'acides gras tels que le stéarate de magnésium, le stéarate de calcium, le stéarate de sodium,
- les cires telles que les cires microcristallines, la cire blanche, la cire de Carnauba, la paraffine,
- les polyoxyéthylène glycols de poids moléculaire élevé, commercialisés sous le nom de CARBOWAX,
- les matières grasses végétales telles que le beurre de karité, l'huile de monoï,
- les esters d'acides gras tels que le myristate d'isopropyle, le cetyl palmitate, le glycéryl stéarate, le palmitate d'isopropyle,
- les alkylméthyle siloxanes tels que le stéaryl diméthicone, le cétyl diméthicone.

On utilisera de préférence au moins 40% en poids, de préférence de 60 à 90 %, de préférence encore de 70 à 80 %, d'excipient(s).

On utilisera de préférence de 0,1 à 60% de matière première aromatique, de préférence encore de 10 à 40%, plus préférentiellement encore de 20 à 30%.

Certains excipients sont particulièrement adaptés à l'obtention d'un effet retard, c'est-à-dire d'une libération retardée de la matière première aromatique. On peut citer non limitativement les acides gras, les esters de glycérol, les huiles hydrogénées, les cires, les alkylméthyl siloxanes et les polyoxyéthylèneglycols estérifiés.

L'homme du métier choisira bien entendu le ou les excipients de telle sorte qu'ils soient inertes vis à vis de la matière première aromatique volatile.

La préparation selon l'invention est particulièrement adaptée aux matières premières aromatiques très volatiles qui sont particulièrement fragiles et difficiles à fixer. C'est le cas notamment du 2,6-diméthyl-7-octèn-2-ol (connu sous la marque Dihydromyrcenol) ou du méthyldihydrojasmonate (connu sous la marque Hedione).

Le 2,6-diméthyl-7-octèn-2-ol ou le méthyldihydrojasmonate seront utilisés de préférence avec un ou plusieurs des excipients suivants : les sels d'acides gras, les esters de glycérol, les huiles hydrogénées, les alkylméthyl siloxanes, les cires, les polyoxyéthylènes estérifiés, les matières grasses végétales.

De manière particulièrement préférée, on utilisera avec le 2,6-diméthyl-7-octèn-2-ol un mélange de cires avec un alkylméthyl siloxane. On préférera plus particulièrement un mélange d'excipients contenant au moins 20% d'un alkylméthyl siloxane.

Le procédé de fabrication des préparations selon l'invention est détaillé ci-après. De manière simplifiée, on mélange la matière première aromatique avec un ou plusieurs excipients fusibles à l'état fondu, de façon à former une masse fondue. Ensuite, la masse fondue est forcée à travers une buse et les microbilles obtenues sont refroidies. Il est indispensable au cours du procédé de ne pas utiliser de températures trop élevées ou pendant trop longtemps, sous peine de voir la matière première aromatique volatile détériorée. Plus précisément, on sait que les matières premières odorantes ne doivent pas être exposées à des températures trop élevées ou à un chauffage trop long (température maximum de 70 à 100°C en fonction de la durée de chauffage pouvant être comprise entre quelques secondes et 4 heures), sous peine d'être altérées et de perdre leur propriétés et donc leurs intérêt. Tout procédé de fabrication devra donc respecter cette limitation.

A la matière première aromatique et à l'excipient ou aux excipients, c'est-à-dire à la masse fondue, il est parfois souhaitable d'ajouter d'autres composants.

Ainsi, on peut ajouter un ou des polymères solubles ou dispersibles dans la masse fondue. Ces polymères vont permettre de favoriser une dissolution maîtrisée et modulable des microbilles lors de leur utilisation dans une composition cosmétique. Il peut s'agir de tout polymère approprié, tel que :
- les dérivés cellulosiques (hydroxypropylcellulose, hydroxypropylméthycellulose, hydroxyéthylcellulose, éthycellulose, carboxyméthylcellulose),
- les résines acryliques,
- les polyvinylacétates,
- les allyl méthacrylates crosspolymères,
- les résines de polyalkylène (éthylène propylène), de polylactique, d'anhydride maléique, de silicone.

On peut également ajouter à la masse fondue un ou plusieurs additifs minéraux qui permettent, avec les excipients, d'accélérer la solidification des microbilles, en particulier lorsque les matières premières aromatiques présentent un phénomène de surfusion. On peut citer à titre d'exemple de tels additifs :
- les silices,
- les oxydes minéraux tels que l'oxyde de titane, l'oxyde de fer,
- les phosphates,
- les carbonates,
- les argiles,
- le talc.

Afin d'améliorer la dispersion ou la solubilité de la matière première aromatique dans le ou les excipients, il est parfois utile d'ajouter un ou plusieurs agents tensioactifs. A titre d'exemple, de tels agents tensioactifs seront choisis parmi les propylène glycol dicaprylate/dicaprate, les esters de sorbitan, les alcools polyéthoxylénés, les glycols tels que la glycérine ou le dipropylène glycol.

Par ailleurs, on pourra également ajouter à la préparation un antioxydant, tel que la vitamine E, et éventuellement un colorant.

Ainsi, la préparation selon l'invention comprend :
- une matière première aromatique volatile ou un mélange de matières premières aromatiques volatiles,
- un ou plusieurs excipients fusibles,
- éventuellement un ou plusieurs polymères dispersibles dans la masse fondue formée par les composants de la préparation,
- éventuellement un ou plusieurs additifs minéraux,
- éventuellement un ou plusieurs agents tensioactifs,
- éventuellement un ou plusieurs antioxydants,
- éventuellement un ou plusieurs colorants.

Comme précisé ci-dessus, le procédé de préparation des microbilles consiste à préparer une masse fondue du ou des excipients ainsi que des éventuels autres composants puis à mélanger cette masse fondue avec la matière première aromatique volatile. Cette masse fondue peut être préparée par fusion séparée des divers constituants suivie de leur mélange avec la matière première aromatique, ou par fusion du mélange des constituants puis mélange avec la matière première aromatique. Les éventuels composants non solubles ou particulièrement volatils sont additionnés à la fin de la fusion de façon à obtenir une masse homogène.

Le choix de la matière première aromatique et des différents composants de la masse fondue est effectué par l'homme du métier en fonction de la compatibilité des constituants, de la viscosité du mélange, de la lipophilie de la matière première aromatique, de la taille particulaire des éventuels composés insolubles, du point de fusion de la matière première aromatique, de la dimension des microbilles désirées, du taux de matière première aromatique à y inclure, du temps de libération de la matière première aromatique désiré et du point de fusion des microbilles désiré.

Les microbilles selon l'invention pourront avoir un point de fusion compris entre 10°C et 90°C, de préférence entre 25°C et 70°C, de préférence encore entre 30°C et 40°C ou 50°C et 60°C. Les conditions de conservation des microbilles seront fonction de leur point de fusion. Des microbilles ayant un point de fusion élevé pourront être conservées à des températures plus élevées que des microbilles ayant un point de fusion bas. A titre d'exemple, des microbilles ayant un point de fusion de 25°C à 30°C seront de préférence conservées et manipulées à une température inférieure à 10°C ou formulées dans un hydrogel.

De préférence, le diamètre des microbilles selon l'invention sera compris entre 5 µm et 5 mm, de préférence encore entre 10 µm et 4 mm, plus préférentiellement encore entre 10 µm et 1500 µm. D'une manière générale, plus le point de fusion des microbilles sera élevé, plus les microbilles seront de préférence petites afin d'exposer à la peau une surface d'échange thermique maximale leur permettant de fondre rapidement.

Plusieurs procédés connus peuvent être mis en oeuvre pour préparer les microbilles selon l'invention. Par exemple, le procédé de fabrication de microbilles décrit dans le brevet FR 2 657 257 peut être utilisé pour préparer les microbilles selon l'invention sous réserve de la limite maximale de température et de durée compatible avec les matières mises en oeuvre. Ce procédé consiste dans une première étape à mélanger le principe actif (matière première aromatique volatile) avec le ou les excipients à l'état fondu, puis à forcer le passage de la masse fondue à travers une buse qui subit une vibration, avant de laisser tomber les microbilles formées dans une tour à contre courant avec un gaz introduit dans le bas de la tour et évacuer en dessous de la buse, les microbilles étant formées dans le bas de la tour.

D'autres procédés de fabrication de microbilles peuvent être utilisés, par exemple la technique du spray cooling, avec la même réserve.

La préparation selon l'invention pourra être utilisée en cosmétique, c'est-à-dire dans une composition pour le corps, la peau ou les cheveux. On pourra ainsi les utiliser dans les compositions cosmétiques suivantes, données à titre d'exemples :
- les produits parfumants, tels que le parfum solide,
- les produits de soin du corps et du visage, tels que lait, crème et hydrogel,
- les produits moussants rincés, tels que shampoing et gel douche,
- les produits conditionneurs, tels que gel capillaire fixant et après shampoing.
- les crèmes solaires.

Elle peut également être utilisée pour formuler des produits ménagers comme par exemple des détergents, des lessives en poudre ou liquides, des assouplissants.

### EXEMPLE 1

Les matières premières très volatiles qui composent les notes Cyclamen, muguet, jasmin, gardénia, pétale de rose et mandarine sont protégées lorsqu'elles sont utilisées dans les microbilles selon l'invention. Elles résistent à un test de 1 mois à 50°C, et la rémanence sur la peau ou le cheveu de ces molécules très légères est améliorée.

### EXEMPLE 2

On prépare des microbilles selon l'invention, en utilisant les composés suivants :
- matière première aromatique volatile ou mélange de matières premières aromatiques volatiles (parfum) : 0,1 à 60 % en poids.
- alkylméthyl siloxanes : au moins 40 % en poids. Il peut s'agir par exemple
   - du DC 2503 Cosmetic Wax de Dow Corning, qui a un point de fusion de 32°C, ou
   - du AMS-C30 Cosmetic Wax de Dow Corning, qui a un point de fusion de 70°C, ou
   - d'un mélange des deux.
- autres composés : 0 à 49,8 % en poids, de préférence 0,1 à 49 % en poids,
- Antioxydant, par exemple BHT ou Oxynex 2004 : 0 à 0,1 % en poids, de préférence 0,01 à 0,10 % en poids,
- Colorants hydrophobes : q.s.

On prépare des microbilles ayant des points de fusion variant entre 25 et 70°C, et un diamètre compris entre 50 µm et 2 mm de la manière suivante : on chauffe les matières fusibles et on ajoute les matières premières aromatiques pour obtenir un mélange totalement liquide. On fait couler le mélange obtenu goutte à goutte dans de l'alcool 96° dénaturé au triéthyl citrate, et on obtient des microbilles.

On peut utiliser une buse de diamètre 1 mm, avec ou sans vibreur, alimentée par un système de pompage thermorégulé relié à une cuve à agitation themorégulée pour conserver le mélange parfum / alkylméthyl siloxane /autres constituants à l'état liquide. Le mélange tombe dans l'alcool et fige pour former des microbilles parfaitement rondes. Le milieu de chute peut être en mouvement et/ou peut être thermorégulé. On obtiendra des microbilles de diamètre compris entre 500 µm et 4 mm en fonction du mode opératoire.

Une fois les microbilles formées, on place l'ensemble microbilles/alcool au frigidaire à 4°C, puis on récupère les microbilles par filtration. Pour éviter les phénomènes de suintement et de collage des microbilles entre elles, on peut ajouter aux microbilles de l'aérosil dont on soufflera l'excédent à l'air froid.

On peut également utiliser un procédé de spray cooling, qui permettra d'obtenir des microbilles de diamètre plus petit, de l'ordre de 50 µm à 500 µm.

### Exemple 2A.

### Composition de la préparation de microbilles selon l'invention

| Composant | Fabricant | % en poids |
|---|---|---|
| Stéaryl diméthicone (DC 2503) | Dow Corning | 74,95 |
| Vitamine E acétate | BASF | 5,00 |
| Parfum (mélange de matières premières aromatiques) | MANE | 20,00 |
| Colorant liposoluble Bleu Covapate W 6763 | Wackherr | q.s. |
| Antioxydant Oxynex (BHT, ascorbyl palmitate, citric acid, glycéryl stéarate, propylène glycol) | Merck | 0,05 |

Les microbilles obtenues sont formées au moyen d'une buse, ont un point de fusion compris entre 25 et 30°C, un diamètre compris entre 800 et 1600 µm. Elles sont conservées et manipulées à des températures inférieures à 10°C ou formulées dans un hydrogel. Le produit fini comprenant les microbilles, c'est-à-dire le gel corporel, pourra être conditionné dans un emballage Airless type Magic Dispenser F de la société WIKO.

Cette préparation solide pourra être utilisée dans un gel pour le corps, dont la composition est la suivante :

| Composant | Fabricant | % en poids |
|---|---|---|
| PHASE A | | |
| Carbomère (Ultrez 10) | Gattefosse | 0,30 |
| Propylène glycol | | 3,50 |
| Eau déminéralisée | | qs 100 |

| PHASE B | | |
|---|---|---|
| Glycérine | | 5,00 |
| Ethanol (alcool 96°) | | 20,00 |
| Allantoïne | Merck | 0,10 |
| Diméthicone copolymère (Abill B 8851 D) | Goldschmidt | 1,00 |
| DMDM Hydantoine (Glydant) | Lonza | 0,20 |

| PHASE C | | |
|---|---|---|
| Microbilles selon tableau ci-dessus | Mane | 1,00 |

| PHASE D | | |
|---|---|---|
| Triéthanolamine | | A pH 5,50 |
| Colorant | Wackherr | qs |
| PPG-7 cétéareth (Eumulgin L) | Henkel | 2,00 |
| Parfum liquide | Mane | 0,30 |

### Exemple 2B.

### Composition de la préparation de microbilles selon l'invention :

| Composant | Fabricant | % en poids |
|---|---|---|
| Stéaryl diméthicone (DC 2503) | Dow Corning | 51,95 |
| C30-45 alkyl méthicone (AMS C-30) | Dow Corning | 25,00 |
| Vitamine E acétate | BASF | 3,00 |
| Parfum (mélange de matières premières aromatiques) | MANE | 20,00 |
| Colorant liposoluble (Brun Covapate W 8760) | Wackherr | q.s. |
| Antioxydant Oxynex (BHT, ascorbyl palmitate, citric acid, glycéryl stéarate, propylène glycol) | Merck | 0,05 |

Les microbilles obtenues sont formées par spray cooling ou prilling, ont un point de fusion compris entre 50 et 57°C, un diamètre compris entre 50 et 500 µm. Elles sont conservées et manipulées à des températures inférieures à 30°C ou formulées dans un hydrogel.

Cette préparation solide pourra être utilisée dans un shampoing ou gel douche.

La composition d'un tel shampoing est donnée ci-après à titre d'exemple :

| Composant | Fabricant | % en poids |
|---|---|---|
| PHASE A | | |
| Carbomère (Carbopol EDT 2001) | Gattefosse | 1,00 |
| Propylène glycol | | 10,00 |
| Eau déminéralisée | | qs 100 |

| PHASE B | | |
|---|---|---|
| TEA-lauryl sulfate (Texapon T42) | Henkel | 15,00 |
| Cocamidopropylbetaine (Tegobetaine HS) | Goldschmidt | 5,00 |
| Ethylène glycol (Emkanol) | | 2,00 |
| DMDM Hydantoine (Glydant) | Lonza | 0,20 |

| PHASE C | | |
|---|---|---|
| Microbilles selon tableau ci-dessus | Mane | 1,00 |

| PHASE D | | |
|---|---|---|
| Triéthanolamine | | A pH 5,50 |
| Colorant | Wackherr | qs |
| Parfum liquide | Mane | 0,30 |

La composition d'un tel gel douche est donnée ci-après à titre d'exemple :

| Composant | Fabricant | % en poids |
|---|---|---|
| PHASE A | | |
| Crosspolymère acrylates/vinyl isodécanoate (Stabilen 30) | 3V Sigma | 0,50 |
| Propylène glycol | | 2,00 |
| Eau déminéralisée | | qs 100 |

| PHASE B | | |
|---|---|---|
| Sodium lauryl sulfate (Texapon N40) | Henkel | 20,00 |
| Cocamidopropylbetaine (Tegobetaine HS) | Goldschmidt | 5,00 |
| Ethylène glycol (Emkanol) | | 2,00 |
| DMDM Hydantoine (Glydant) | Lonza | 0,20 |

| PHASE C | | |
|---|---|---|
| Microbilles selon tableau ci-dessus | Mane | 1,50 |

| PHASE D | | |
|---|---|---|
| Hydroxyde de sodium (solution aqueuse 10%) | | A pH 7 |
| Colorant | Wackherr | qs |
| Parfum liquide | Mane | 0,70 |

### EXEMPLE 3

On réalise un test afin d'évaluer la rémanence de microbilles selon l'invention. Le test est effectué sur un panel expert de 20 personnes, constitué de parfumeurs et évaluateurs.

On applique sur chacune des 20 personnes le même parfum sous deux formes différentes : 0,015 g de parfum liquide sur un avant-bras, et des microbilles selon l'invention sur l'autre avant-bras. L'évaluation de la diminution de l'odeur (en %) est effectuée par chaque personne à différents temps (en heures) après l'application : t=0, 1h, 2h et 5h.

Les moyennes de ces estimations sont représentées sur l'histogramme ci-après, en blanc pour le parfum liquide, en grisé pour les microbilles.

On observe nettement que la forme microbilles devient nettement plus efficace que le parfum liquide 5h après l'application sur la peau, ce qui prouve la rémanence élevée des microbilles.

### EXEMPLE 4

On prépare des microbilles selon l'invention en utilisant les composés suivants :

| Composant | Fabricant | % en poids |
|---|---|---|
| Alkylméthyl siloxanes (DC 2503) | Dow Corning | 45,00 |
| Cire de jojoba hydrogénée | Jan Dekker | 35,00 |
| Fragrance : parfum Axal | Mane | 20,00 |

Les microbilles, obtenues selon le procédé de l'invention, ont un point de fusion compris entre 50°C et 65°C avec un diamètre compris entre 5 µm et 40 µm.

Ces microbilles sont formulées dans un liquide assouplissant pour linge non concentré, dont la composition B est donnée ci-après :

| Composant | Fabricant | % en poids |
|---|---|---|
| Dihydrogenated palmoylethyl hydroxyethylmonium methosulfate (Rewoquat WE 28) | Witco | 5,65 |
| Eau déminéralisée | | qsp 100 |
| Microbilles Axal | Mane | 1,50 |

Un autre assouplissant est préparé en remplaçant les microbilles par 0,3 % en poids de fragrance Axal sous forme liquide (composition A).

On réalise un test de rémanence parfum pour les deux formes de présentation, correspondant aux deux compositions d'assouplissant A et B. Le test est effectué sur un panel expert de 20 personnes, constitué de parfumeurs et évaluateurs.

Il est effectué avec des lingettes en coton qui ont subi un cycle de lavage classique en machine à laver le linge réglée à une température de 45°C. Chaque machine à laver contient 2 kg de linge, et la charge en assouplissant (composition A ou B) est de 100 g.

A l'issu du cycle de lavage, chaque personne membre du panel désigne la lingette ayant l'odeur de parfum la plus puissante. Les évaluations se font à t=0 (linge humide), puis t= 24, 48, et 96 heures (linge sec).

Les résultats sont portés sur l'histogramme ci-après, en blanc pour le parfum liquide (composition A), en grisé pour les microbilles (composition B) :

On observe nettement que le parfum sous forme de microbilles devient plus efficace (rémanence olfactive plus importante) que le parfum liquide à partir du temps t=24h, et ce phénomène se poursuit à t=48h et t=96h.

La présente invention permet donc de favoriser la fixation de molécules aromatiques, en particulier de molécules utilisées en note de tête dans l'industrie de la parfumerie, et la protection de ces molécules utilisées seules ou en mélange dans un milieu qui peut être riche en matières actives inhibitrices, tel que le peroxyde d'hydrogène et le dihydroxyacétone.

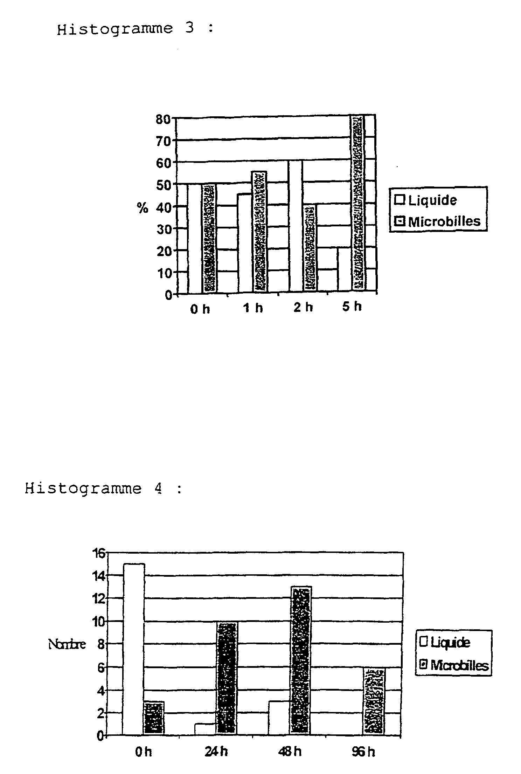

## Revendications

1. Préparation parfumée solide à rémanence élevée se présentant sous forme de microbilles de composition en masse homogène et comprenant une matière première aromatique volatile, ou un mélange de matières premières aromatiques volatiles, et un ou plusieurs excipients fusibles.

2. Préparation selon la revendication 1, comprenant en outre l'un au moins des composants suivants :
- un ou plusieurs polymères dispersibles dans la masse fondue des composants de 1a préparation,
- un ou plusieurs additifs minéraux,
- un ou plusieurs agents tensioactifs,
- un ou plusieurs antioxydants,
- un ou plusieurs colorants.

3. Préparation selon l'une ou l'autre des revendications 1 et 2, comprenant au moins 40% en poids, de préférence de 60 à 90%, de préférence encore de 70 à 80%, d'excipient (s).

4. Préparation selon l'une quelconque des revendications 1 à 3, comprenant de 0,1 à 60 % en poids de matière première aromatique, de préférence de 10 à 40%, de préférence encore de 20 à 30%.

5. Préparation selon l'une quelconque des revendications 1 à 4, dans laquelle la ou l'une des matières premières aromatiques volatiles est choisie parmi les hydrocarbures aromatiques, terpéniques et/ou sesquiterpéniques ; les alcools aromatiques ; les alcools non aromatiques primaires ou secondaires ou tertiaires, saturés ou insaturés, cycliques ou acycliques ; les aldéhydes ; les phénols ; les acides carboxyliques ; les éthers et acétals aromatiques et/ou non aromatiques sous leur forme acyclique ou cyclique ; les hétérocycles contenant l'atome d'azote ; les cétones ; les sulfures, disulfures et mercaptans aromatiques ou non aromatiques, les huiles essentielles.

6. Préparation selon l'une quelconque des revendications 1 à 5, dans laquelle le ou les excipients fusibles sont choisis parmi les alcools gras ; les acides gras ; les esters de glycérol ; les huiles hydrogénées ; les sels d'acides gras ; les cires ; les polyoxyéthylènes glycols de poids moléculaire élevé ; les matières grasses végétales ; les esters d'acides gras ; les alkylméthyl siloxanes.

7. Préparation selon la revendication 6, comprenant au moins un alkylméthyl siloxane en tant qu'excipient.

8. Préparation selon la revendication 7, dans laquelle l'alkylméthyl siloxane est présent en une quantité d'au moins 20 % en poids

9. Préparation selon l'une quelconque des revendications 1 à 8, dans laquelle la ou l'une des matières premières aromatiques est le 2,6-diméthyl-7-octèn-2-ol ou le méthyldihydrojasmonate.

10. Préparation selon l'une quelconque des revendications 1 à 9, se présentant sous forme de microbilles d'un diamètre compris entre 5 µm et 5 mm, de préférence entre 10 µm et 4 mm, et plus préférentiellement encore entre 10 µm et 1500 µm.

11. Préparation selon l'une quelconque des revendications 1 à 10, dans laquelle les microbilles présentent un point de fusion compris entre 10°C et 90°C, de préférence entre 25°C et 70°C, de préférence encore entre 25°C et 30°C ou 50°C et 60°C.

12. Composition cosmétique ou produit ménager comprenant une préparation selon l'une quelconque des revendications 1 à 11.

13. Composition cosmétique selon la revendication 12, choisie parmi les produits parfumants, tels que le parfum solide ; les produits de soin du corps et du visage, tels que lait, crème et hydrogel ; les produits moussants rincés, tels que shampoing et gel douche ; les produits conditionneurs, tels que gel capillaire fixant et après shampoing ; les crèmes solaires.

14. Produit ménager selon la revendication 12, choisi parmi les produits détergents, les produits assouplissants, les produits de traitement des textiles.

15. Procédé de fabrication d'une préparation selon l'une quelconque des revendications 1 à 11, **caractérisé en ce que** l'on mélange la matière première aromatique avec le ou les excipients à l'état fondu et les autres composés de façon à obtenir une masse fondue, on force le passage de la masse fondue à travers une buse et on refroidit les microbilles obtenues de façon à obtenir des produits solidifiés, la température atteinte au cours du procédé étant de 70 à 100°C en fonction de la durée de chauffage comprise entre quelques secondes et 4 heures.

## Patentansprüche

1. Feste Parfümzusammensetzung mit erhöhter Remanenz in Form von Mikrokugeln, die eine homogene Gewichtzusammensetzung haben und einen flüchtigen aromatischen Grundstoff oder ein Gemisch aus flüchtigen aromatischen Grundstoffen sowie eine oder mehrere schmelzbare Trägermassen enthalten.

2. Parfümzusammensetzung nach Anspruch 1, die zudem mindestens einen der folgenden Bestandteile enthält:
- ein oder mehrere in der Schmelze der Präparatbestandteile dispergierbare Polymere,
- einen oder mehrere mineralische Zusatzstoffe,
- einen oder mehrere Tenside,
- ein oder mehrere Antioxidantien,
- einen oder mehrere Farbstoffe.

3. Parfümzusammensetzung entweder nach Anspruch 1 oder Anspruch 2, enthaltend mindestens 40 Gewichtsprozent, vorzugsweise 60 bis 90%, noch besser 70 bis 80%, an Trägermasse(n).

4. Parfümzusammensetzung nach einem der Ansprüche 1 bis 3, enthaltend 0,1 bis 60 Gewichtsprozent, vorzugsweise 10 bis 40%, und besser 20 bis 30% an aromatischem Grundstoff.

5. Parfümzusammensetzung nach einem der Ansprüche 1 bis 4, bei welcher der oder einer der flüchtigen aromatischen Grundstoffe gewählt wird aus aromatischen Kohlenwasserstoffen, Terpen-Kohlenwasserstoffen und/oder Sesquiterpen-Kohlenwasserstoffen; aromatischen Alkoholen; zyklischen oder azyklischen, gesättigten oder ungesättigten, primären, sekundären oder tertiären nicht aromatischen Alkoholen; Aldehyden; Phenolen; Karboxylsäuren; aromatischen und/oder nicht aromatischen Ethern und Acetalen in ihrer azyklischen oder zyklischen Form; heterozyklischen Verbindungen mit Stickstoffatom; Ketonen; Sulfiden, Bisulfiden und aromatischen oder nicht aromatischen Merkaptanen, essentiellen Ölen.

6. Parfümzusammensetzung nach einem der Ansprüche 1 bis 5, bei welcher die schmelzbare Trägermasse oder die schmelzbaren Trägermassen ausgewählt werden aus: Fettalkoholen; Fettsäuren; Glyzerinestern; hydrierten Fetten; Fettsäuresalzen; Wachsen; Polyoxiäthylenglykole mit höherem Molekülargewicht; pflanzlichen Fetten, Fettsäureestern; Alkylmethylsiloxanen.

7. Parfümzusammensetzung nach Anspruch 6, die mindestens ein Alkylmethylsiloxan als Trägermasse enthält.

8. Parfümzusammensetzung nach Anspruch 7, bei der das Alkylmethylsiloxan in einer Menge von mindestens 20 Gewichtsprozenten vorliegt.

9. Parfümzusammensetzung nach einem der Ansprüche 1 bis 8, bei dem der oder einer der aromatischen Grundstoffe 2,6-Dimethyl-7-Okten-2-ol oder Methyldihydrojasmonat ist.

10. Parfümzusammensetzung nach einem der Ansprüche 1 bis 9, welche in Form von Mikrokugeln mit einem Durchmesser von zwischen 5 µm und 5 mm, vorzugsweise zwischen 10 µm und 4 mm und noch besser zwischen 10 µm und 1500 µm vorliegt.

11. Parfümzusammensetzung nach einem der Ansprüche 1 bis 10, bei welcher die Mikrokugeln einen Schmelzpunkt zwischen 10°C und 90°C, vorzugsweise zwischen 25°C und 70°C, besser noch zwischen 25°C und 30°C oder 50°C und 60°C aufweisen.

12. Kosmetische Zusammensetzung oder Haushaltsprodukt, enthaltend eine Parfümzusammensetzung nach einem der Ansprüche 1 bis 11.

13. Kosmetische Zusammensetzung nach Anspruch 12 als Auswahl von parfümierenden Produkten wie Feststoffparfüm; Körper- und Gesichtspflegeprodukten wie Milch, Creme und Hydrogel; abspülbaren, schäumenden Produkten wie Shampoo und Duschgel; Konditionierprodukten wie Haarfestigergel und Haarspülungsprodukte; Sonnencremes.

14. Haushaltsprodukt nach Anspruch 12 als Auswahl von Waschmitteln, Weichspülern, Textilbehandlungsprodukten.

15. Verfahren zur Herstellung einer Parfümzusammensetzung nach einem der Ansprüche 1 bis 11, **dadurch gekennzeichnet, dass** der aromatische Grundstoff mit der Trägermasse oder den Trägermassen in geschmolzenem Zustand und den übrigen Verbindungen derart gemischt wird, dass man eine Schmelze erhält, dass die Schmelze durch eine Düse gepresst wird und die entstandenen Mikrokugeln so abgekühlt werden, dass man feststoffliche Erzeugnisse erhält, wobei die im Verlaufe des Verfahrens erreichte Temperatur 70 bis 100°C beträgt, in Abhängigkeit von der Dauer der Erhitzung, welche zwischen einigen Sekunden und 4 Stunden liegt.

## Claims

1. Solid fragranced preparation with increased persistence in the form of microbeads of a composition homogeneous in mass and comprising a volatile aromatic raw material or a mixture of volatile aromatic raw materials, and one or more meltable excipients.

2. Preparation as claimed in claim 1, also comprising at least one of the following components:
- one or more polymers that are dispersible in the molten mass of the components of the preparation,
- one or more mineral additives,
- one or more surfactants,
- one or more antioxidants,
- one or more colorants.

3. Preparation as claimed in either of claims 1 and 2, comprising at least 40% by weight, preferably from 60% to 90% and more preferably from 70% to 80%, of excipient(s).

4. Preparation as claimed in any one of claims 1 to 3, comprising from 0.1% to 60% by weight of aromatic raw material, preferably from 10% to 40% and more preferably from 20% to 30%.

5. Preparation as claimed in any one of claims 1 to 4, in which the volatile aromatic raw material(s) is (are) chosen from aromatic, terpenic and/or sesquiterpenic hydrocarbons; aromatic alcohols; primary, secondary or tertiary, saturated or unsaturated, cyclic or acyclic nonaromatic alcohols; aldehydes; phenols; carboxylic acids; aromatic and/or nonaromatic ethers and acetals in their acyclic or cyclic form; heterocycles containing a nitrogen atom; ketones; aromatic or nonaromatic sulfides, disulfides and mercaptans, and essential oils.

6. Preparation as claimed in any one of claims 1 to 5, in which the meltable excipient(s) is (are) chosen from fatty alcohols; fatty acids; glycerol esters; hydrogenated oils; fatty acid salts; waxes; polyoxyethylene glycols of high molecular weight; plant fats; fatty acid esters; alkyl methyl siloxanes.

7. Preparation as claimed in claim 6, comprising at least one alkylmethyl siloxane as excipient.

8. Preparation as claimed in claim 7, in which the alkylmethyl siloxane is present in an amount of at least 20% by weight.

9. Preparation as claimed in any one of claims 1 to 8, in which the aromatic raw material(s) is(are) 2,6-dimethyl-7-octen-2-ol or methyl dihydrojasmonate.

10. Preparation as claimed in any one of claims 1 to 9, which is in the form of microbeads with a diameter of between 5 µm and 5 mm, preferably between 10 µm and 4 mm and even more preferably between 10 µm and 1 500 µm.

11. Preparation as claimed in any one of claims 1 to 10, in which the microbeads have a melting point of between 10°C and 90°C, preferably between 25°C and 70°C and more preferably between 25°C and 30°C or 50°C and 60°C.

12. Cosmetic composition or household product comprising a preparation as claimed in any one of claims 1 to 11.

13. Cosmetic composition as claimed in claim 12, chosen from fragrancing products, such as solid perfume; bodycare and facialcare products, such as milk, cream and hydrogel; rinse-out foaming products, such as shampoo and shower gel; conditioning products, such as fixing hair gel and conditioner; sun creams.

14. Household product as claimed in claim 12, chosen from detergent products, softening products and textile treatment products.

15. Process for manufacturing a preparation as claimed in any one of claims 1 to 11, **characterized in that** the aromatic raw material is mixed with the molten excipient(s) and the other components so as to obtain a molten mass, the molten mass is forced through a nozzle and the microbeads obtained are cooled so as to obtain solidified products, the temperature reached during the process being from 70 to 100°C depending on the duration of heating, which is between a few seconds and 4 hours.
